(19) Europäisches Patentamt
European Patent Office
Office européen des brevets

(11) Publication number: **0 325 723 A2**

# (12) EUROPEAN PATENT APPLICATION

(21) Application number: **88119491.4**

(22) Date of filing: **23.11.88**

(51) Int. Cl.4: **G01N 33/86 , G01N 33/577 , G01N 33/563 , G01N 33/546**

The title of the invention has been amended (Guidelines for Examination in the EPO, A-III, 7.3).

(30) Priority: **03.12.87 JP 306730/87**

(43) Date of publication of application: **02.08.89 Bulletin 89/31**

(84) Designated Contracting States: **AT BE CH DE ES FR GB GR IT LI LU NL SE**

(71) Applicant: **DAIICHI PURE CHEMICALS CO. LTD.**
**13-5, Nihonbashi 3-chome**
**Chuo-ku Tokyo(JP)**

(72) Inventor: **Sakai, Yasuo c/o Daiichi Pure Chemicals Co., Ltd.**
**5-5-12, Narihira Sumida-ku**
**Tokyo(JP)**
Inventor: **Takei, Fujio c/o Daiichi Pure Chemicals Co., Ltd.**
**5-5-12, Narihira Sumida-ku**
**Tokyo(JP)**
Inventor: **Matsumoto, Takashi c/o Daiichi Pure Chem. Co., Ltd**
**5-5-12, Narihira Sumida-ku**
**Tokyo(JP)**
Inventor: **Maeda, Makiko c/o Daiichi Pure Chemicals Co., Ltd.**
**5-5-12, Narihira Sumida-ku**
**Tokyo(JP)**

(74) Representative: **Wächtershäuser, Günter, Dr.**
**Tal 29**
**D-8000 München 2(DE)**

## (54) Method for the determination of blood clotting factors and reagent for use therein.

(57) A method for the determination of a blood clotting factor is disclosed. The method comprises reacting a sample containing a blood clotting factor with an insoluble carrier reagent which is sensitized by a monoclonal antibody specific to the blood clotting factor, and comparing the amount of non-agglutinated particles with the amount of agglutinated particles produced. A reagent for blood clotting factor determination comprising an insoluble carrier having a particle size of 0.5 to 2 µm which is sensitized with monoclonal antibody specific to the blood clotting factor is also disclosed. The method and the reagent provide a simple and speedy determination of a clotting factors in living bodies, and are useful in a variety of fields, including medical treatment and clinical inspection.

EP 0 325 723 A2

# METHOD FOR THE DETERMINATION OF BLOOD CLOTTING FACTORS AND REAGENT FOR USE IN THE DETERMINATION

## BACKGROUND OF THE INVENTION

### Field of the Invention:

This invention relates to a method and a reagent for detecting and quantitatively analyzing, with the use of monoclonal antibody, a blood clotting factor contained in a sample.

### Description of the Background:

A number of factors are known to be involved in the phenomenon of blood clotting and fibrinolysis. Determination of these factors is essential for the accurate diagnosis and therapy of diseases relating to the blood. For instance, a cross-linked fibrinogen degradation product (hereinafter abbreviated as "FDP"), a type of blood clotting factor, is known to come to abnormally exist in blood and urine, when a subject is in the condition of hypercoagulability or hyper-fibrinolysis, or when he receives surgical treatment. The cause of their production in a living body is considered to be the transformation of fibrinogen into a stabilized fibrin by the action of thrombin or the like, followed by cleavage of the stabilized fibrin by the action of plasmin or the like.

Determination of FDP, therefore, is very important for the detection of blood clotting or fibrinolysis in a living body, and knowing the result of that determination precisely and speedily is clinically imperative.

As is well known the reaction involving blood clotting or fibrinolysis comes to an end within a very short period of time, e.g. within 30 minutes. The phenomenon of blood clotting or fibrinolysis has a high probability of bringing about highly critical conditions to a living body. Thus, from a clinical therapy aspect incorrect or imprecise determination, delayed determination, e.g. taking one hour or longer, or determination requiring a special technique is not permissible and, under some circumstances, could bring about no result.

In the past, blood coagulation has been quantitatively determined through measuring the dotting time for fibrin clot formation. There has been, however, no simple method for the quantitative determination providing a sufficient sensitivity for individual clotting factors. Although synthetic peptide substrates having a specificity for enzymes in living bodies have recently been introduced, these substrates are expensive and require a sophisticated technique for the measurement. There is an established method utilizing radio isotope. This method also requires the use of a special facility and is not free from the problem of the pollution caused by waste materials. The method has not been accepted because of these reasons.

Although quantitative determination of a blood clotting factor, for instance of FDP, has been desired in many of the past cases, unavailability of a suitable technique for the determination, the necessity for a longer period of time, or the requirement of an inexpensive, special device, and like factors have been stumbling blocks to the quantitative determination. Thus, blood clotting factor determination has only been qualitative in most of the past cases.

For instance, the latex agglutination test on a slide plate by the use of latex particles is judged with the naked eye. This inevitably leaves the judgment to the discretion of the person observing and renders the determination qualitative. The enzymatic enzyme immunoassay for the quantitative analysis of FDP (Japanese Patent Laid-open No. 233,553/1985) takes considerable time and requires a certain technique for the determination. As another method, the nephelometric method for quantitative determination with the use of a latex can be considered. This method, however, is liable to sustain a non-specific reaction. Moreover, since the method usually relies upon a colorimetric technique for the determination, either use of a blank sample is necessary or changes in absorbance during the reaction must be detected. This requires a special measuring device for a precise determination.

The inventors have undertaken extensive studies in order to to overcome the drawbacks existing in conventional methods, and to develop a method for speedily determining a clotting factor. As a result, the inventors have found that the use of a monoclonal antibody specific to a blood clotting factor and an insoluble carrier reagent to produce an agglutination, and the comparison of the amounts of non- agglutinated particles and agglutinated particles ensures an easy means for the quantitative determination of a blood clotting factor. The finding has led to the completion of this invention.

## SUMMARY OF THE INVENTION

Accordingly, an object of this invention is to provide a method for the determination of blood clotting factor comprising reacting a sample containing a blood clotting factor with an insoluble carrier reagent which is sensitized by a monoclonal antibody specific to the blood clotting factor, and comparing the amount of non-agglutinated particles with the amount of agglutinated particles produced.

Another object of the present invention is to provide a reagent for blood clotting factor determination comprising an insoluble carrier having a particle size of 0.5 to 2 µm which is sensitized with monoclonal antibody specific to the blood clotting factor.

Other objects, features and advantages of the invention will hereinafter become more readily apparent from the following description.

## BRIEF DESCRIPTION OF THE DRAWINGS

Figure 1 shows a calibration curve determined for FDP using monoclonal antibody 03202.

Figure 2 shows the correlation between the method according to the present invention and enzyme immunological method.

Figure 3 shows another calibration curve determined for FDP using monoclonal antibody 03204.

Figure 4 shows the calibration curve determined for β-TG.

## DETAILED DESCRIPTION OF THE INVENTION AND PREFERRED EMBODIMENTS

Monoclonal antibodies used in the present invention can be prepared according to a conventional method. Beside FDP, protein C, β-TG, AT III, PLG, $\alpha_2$P1, thrombin, FX III, or the like can be used as a blood clotting factor. These monoclonal antibodies can be used after they are selected by conventional method. It is preferable, however, for the purpose of avoiding interference related to complements, thus reducing the occurrence of non-specific reactions, to convert these into $F(ab')_2$ or Fab through digestion by the use of pepsin or papain. Given as examples of monoclonal antibodies having specificity for a blood clotting factor are the monoclonal antibody against FDP (Japanese Patent Laid-open No. 166,698/1985), and the like.

Examples of insoluble carrier particles used in this invention include polyamide-type, polyvinylidene chloride-type, polyvinyl chloride-type, acryl-type, polyvinyl acetate-type, polystyrene-type, polypropylene-type, polyepoxy-type, urethane-type, polyethylene-type, agarose-type chitosan-type, pullulan-type polymer particles as well as other types of polymer particles. It is desirable that these insoluble carrier particles contain a certain proportion of an acid group such as carboxyl group, sulfonic acid group, or the like. A preferable method for preparing these types of polymers is to copolymerize a monomer having no acid group and a monomer having an acid group. Specifically, those having a degree of denaturing with an acid group in the range of 0.1 to 10% is preferably. Given as examples of such insoluble carrier particles which are commercially available are SFL-140L series products, SFL-155L series products, SFL-200L series products, and SFL-140S series products, all manufactured by Japan Synthetic Rubber Co., Ltd.; N-series products manufactured by Sekisui Chemical Co., Ltd.; carboxy-denatured latex particles manufactured by Dow Chemical Co.; K-series latex particles manufactured by Rhone-Poulenc Co.; and the like. Among these insoluble carrier particles those having a particle size preferably of 0.5 to 2 µm, and particularly of 0.6 to 1.2 µm, can be used.

Sensitization of insoluble carrier particles may be performed according to a conventional method. For instance, it can be performed by a physical adsorption method which comprises adding a monoclonal antibody buffer solution to a suspension of insoluble carrier particles, allowing the solution to stand for a duration of 1 hour to overnight, and eliminating the non-sensitized monoclonal antibody by a suitable means such as centrifugation. For the practice of the present invention, however, in order to achieve high sensitivity nd to improve durability and stability of the reagent, the use of a chemical covalent bonding method which comprises sensitizing an insoluble carrier with a monoclonal antibody using a known bi-crosslinking reagent or a bonding agent is desirable. The generally applicable concentration of monoclonal antibody used to sensitize insoluble carrier particles is approximately 25 to 300 µg/ml and that of insoluble carrier particles is in the range of 0.2 to 2%. These concentrations, however, may be varied depending on the type and concentration of clotting agent to be measured, the type and characteristics of monoclonal antibody employed, as well as the property, particle size, and the degree of denaturing with an acid, of the insoluble carrier.

The insoluble carrier sensitized with a monoclonal antibody according to one of the above methods, such a carrier being hereinafter referred to as "insolubilized antibody reagent," is treated with bovine serum albumin (hereinafter abbreviated as "BSA") or a surface active agent of various kinds as required, and is stored as a suspension in a buffer solution or as a lyophilized product.

Determination of a blood clotting factor according to the present invention is performed by adding a sample containing a blood clotting factor to be detected to an insolubilized antibody reagent, incubating the mixture, and measuring for comparison the amounts of non-agglutinated particles and agglutinated particles produced. Plasma, serum, body fluid, or the like can be used as a sample. Incubation is carried out at a temperature of 20 to45 °C for 3 to 30 minutes. The amounts of non-agglutinated particles and agglutinated particles can be determined by counting, by means of a laser scattering method or the like, the number of particles having a particle size corresponding to the insolubilized antibody reagent and the number of particles having a particle size corresponding to the agglutinated particles produced. More specifically, a calibration curve is prepared in advance from the ratio (reaction ratio) of agglutinated particles to non-agglutinated particles produced using a clotting factor sample of known concentration. This calibration curve is then used as a standard for determining the amount of the clotting factor in a sample.

When a conventional type of particle measurement device utilizing an electric resistance method based on the Coulter principle is used, an orifice bore for apertures of 20 to 50 µm is suitable. Precise determination of clotting factors is possible using this type of particle measurement device by the application of the method of this invention.

Major advantages of this invention are: (i) it does not require the use of a defibrinogen, (ii) it can provide a highly sensitive measurement, (iii) the procedure is very simple requiring a very short time period for the measurement; less than 30 minutes starting from blood sampling, (iv) the method can exclude any non-specific reaction, e.g, it is free from the influences due to a rheumatoid factor.

Thus, the method provides a simple and speedy determination of clotting factors in living bodies, and is useful in a variety of the fields, including medical treatment and clinical inspection.

Other features of the invention will become apparent in the course of the following description of the exemplary embodiments which are given for illustration of the invention and are not intended to be limiting thereof.

## EXAMPLES

### Example 1

Measurement of FDP using monoclonal antibody 03202:

(1) Sensitization of insoluble carrier with monoclonal antibody:

A 10% polystyrene latex suspension having a 1.0 µm particle size (SFL-140L manufactured by Japan Synthetic Rubber Co., Ltd.) was diluted to a 10-fold volume using a 0.05 M glycin buffer (pH 8.0). The diluted suspension was mixed with an equivalent amount of a 100 µg/ml FDP monoclonal antibody (monoclonal antibody 03202 described in Japanese Patent Laid-open No.166,698/1985) and the mixture was allowed to stand for 2 hours at 4 °C. Then, the same buffer solution containing 0.5% BSA in an amount of 1 part by volume per 100 parts by volume of the mixture was added. After having been allowed to stand for 2 hours, this mixture was subjected to centrifugation to wash out and eliminate non-sensitized monoclonal antibodies. The residue was ultimately made into a suspension containing 0.2% of BSA and 0.1% of $NaN_3$ in the same 0.05 M glycin buffer.

(2) Measurment of FDP:

(i) 50 µl of a standard FDP with a known concentration and 50 µl of the insolubilized antibody reagent prepared in (1) above were mixed and the mixture was incubated at 37 °C for 10 minutes. Upon termination of the reaction by the addition of a buffer solution, the mixture was diluted to a 100- to 500-fold volume. The particle size distribution of the agglutinated reaction product thus produced was measured according to an electrical resistance method based on the Coulter principle using a Coulter counter ZBI & c-1000.

(ii) The particle size distribution data of the agglutinated reaction product obtained in (i) above was transmitted to a PC9801 computer (manufactured by Nippon Electric Co., Ltd.) which was connected to the Coulter counter ZBI & c-1000. The ratio by volume of non-agglutinated monoclonal antibody sensitized particles to agglutinated monoclonal antibody sensitized particles was calculated to obtain the reaction ratio.

(iii) A calibration curve was prepared by plotting the reaction ratio obtained from the standard FDP along the abscissa and the FDP concentration of the standard along the ordinate. The curve is shown in Fig. 1.

### Example 2

FDP concentrations were measured on various samples according to the method described in Example 1 and the enzyme immunological method described in Japanese Patent Laid-open No. 233,553/1985 to determine the correlation of the two methods. The results are shown in Fig. 2, which demonstrats that the correlation of the FDP concentration values obtained by the application of the method of this invention and those obtained according to the enzyme immunological method described in japanese Patent Laid-open No. 233,553/1985 is very close ($\gamma$ = 0.809).

Example 3

Measurement of FDP using monoclonal antibody 03204:

(1) Sensitization of insoluble carrier with monoclonal antibody:

An insolubilized FDP antibody reagent was prepared in the same manner as in Example 1 except that polystyrene latices having a 0.8 $\mu$m particle size and the monoclonal antibody 03204 described in Japanese Patent Laid-open No.166,698/1985 were used.

(2) Measurement of FDP:

A calibration curve was prepared to determine the amount of FDP by mixing 50 $\mu$l of a standard FDP or 50 $\mu$l a sample with 50 $\mu$l of the insolubilized antibody reagent prepared in (1) above and following the same manner as in (2) (i) through (iii) of Example 1. The results are shown in Fig. 3.

Example 4

Measurement of $\beta$-thromboglobulin ($\beta$-TG) using monoclonal antibodies 11211 and 11224:

(1) Sensitization of insoluble carrier with monoclonal antibody:

A 10% polystyrene latex suspension having a 1.0 $\mu$m particle size (manufactured by Japan Synthetic Rubber Co., Ltd.) was diluted to a 10-fold volume using a 0.05 M glycin buffer (pH 8.0). The diluted suspension was mixed with each 100 $\mu$g/ml $\beta$-TG monoclonal antibody, designated as 11211 and 11224. Each mixture was allowed to stand for 2 hours at an ambient temperature. Then, each was mixed with an equivalent amount of the same glycin buffer solution containing 0.5% BSA and allowed to stand for 2 hours at 4° C. The mixture was subjected to centrifugation to wash out and eliminate non-sensitized monoclonal antibodies. The residue was ultimately made into a suspension containing 0.2% of BSA and 0.1% of $NaN_3$ in the same 0.05 M glycin buffer. To each of the suspensions thus prepared the same amount of each of the monoclonal antibody sensitized particles was added and mixed. The mixtures were stored at 4° C.

(2) Measurement of $\beta$-TG:

(i) 50 $\mu$l of a standard $\beta$-TG having a known concentration and 50 $\mu$l of the insolubilized antibody particles prepared in (1) above were mixed and the mixture was incubated at 37° C for 10 minutes. Upon termination of the reaction by the addition of a termination solution, the mixture was diluted to a 200-fold volume. The particle size distribution of the agglutinated reaction product thus produced was measured according to an electrical resistance method based on the Coulter principle using a Coulter counter ZBI & c-1000.

(ii) The particle size distribution data of the agglutinated reaction product obtained in (i) above was transmitted to a PC9801 computer (manufactured by Nippon Electric Co., Ltd.) which was connected to the Coulter counter ZBI & c-1000. The ratio by volume of non-agglutinated monoclonal antibody sensitized particles to agglutinated monoclonal antibody sensitized particles was calculated to obtain the reaction ratio.

(iii) A calibration curve was prepared by plotting the reaction ratio obtained from the standard $\beta$-TG along the abscissa and the $\beta$-TG concentration of the standard along the ordinate. The curve is shown in Fig. 4.

Obviously, numerous modifications and variations of the present invention are possible in light of the above teachings. It is therefore to be understood that the scope of the appended claims, the invention may be practiced otherwise than as specifically described herein.

**Claims**

1. A method for the determination of a blood clotting factor comprising:
reacting a sample containing a blood clotting factor with an insoluble carrier reagent which is sensitized with monoclonal antibody specific to the blood clotting factor, and
comparing the amount of non-agglutinated particles and the amount agglutinated particles produced.

2. The method according to Claim 1, wherein said monoclonal antibody is a fraction $F(ab')_2$ or a fraction Fab.

3. The method according to Claim 1, wherein said sample is a blood plasma, a serum, or a body fluid.

4. The method according to Claim 1, wherein said insoluble carrrier particles have a particle size of 0.5 to 2 µm and surfaces thereof are covered with an acid group.

5. The method according to Claim 1, wherein an electric resistance method or a laser scattering method is employed to compare the amount of non-agglutinated particles with the amount of agglutinated particles.

6. A reagent for blood clotting factor determination comprising an insoluble carrier having a particle size of 0.5 to 2 µm which is sensitized with monoclonal antibody specific to the blood clotting factor.

7. the reagent according to Claim 6, wherein said monoclonal antibody is a fraction $F(ab')_2$ or a fraction Fab.

FIG. 1

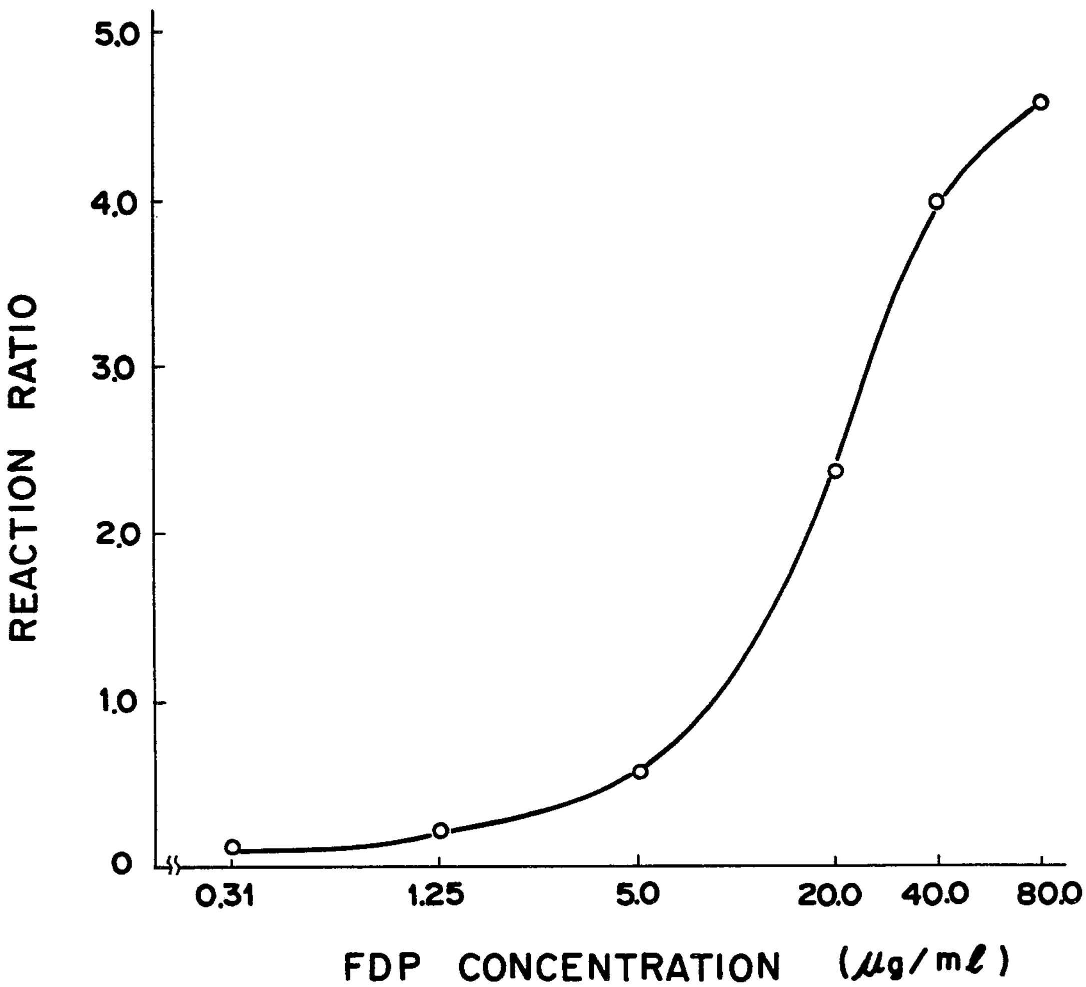
5.0
4.0
3.0
2.0
1.0
0
REACTION RATIO
0.31
1.25
5.0
20.0
40.0
80.0
FDP CONCENTRATION (μg/mℓ)

FIG. 2

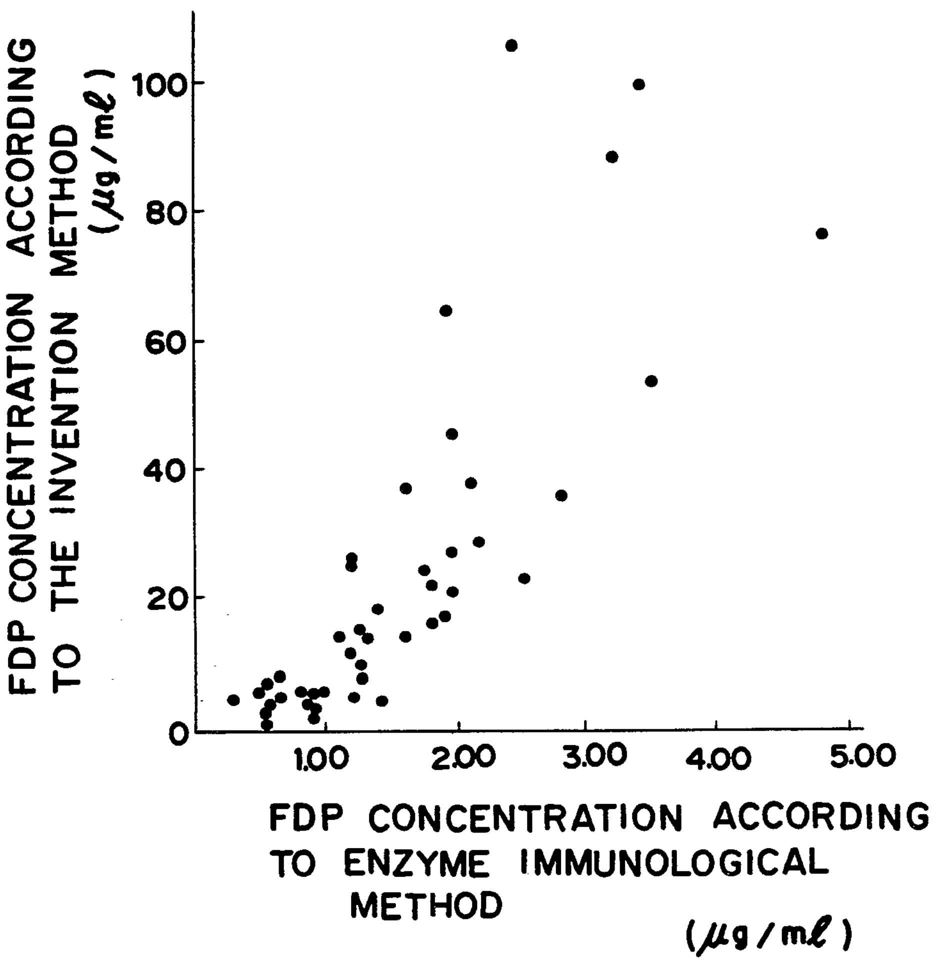
FDP CONCENTRATION ACCORDING TO THE INVENTION METHOD
(μg/mℓ)
100
80
60
40
20
0
1.00
2.00
3.00
4.00
5.00
FDP CONCENTRATION ACCORDING TO ENZYME IMMUNOLOGICAL METHOD
(μg/mℓ)

FIG. 3

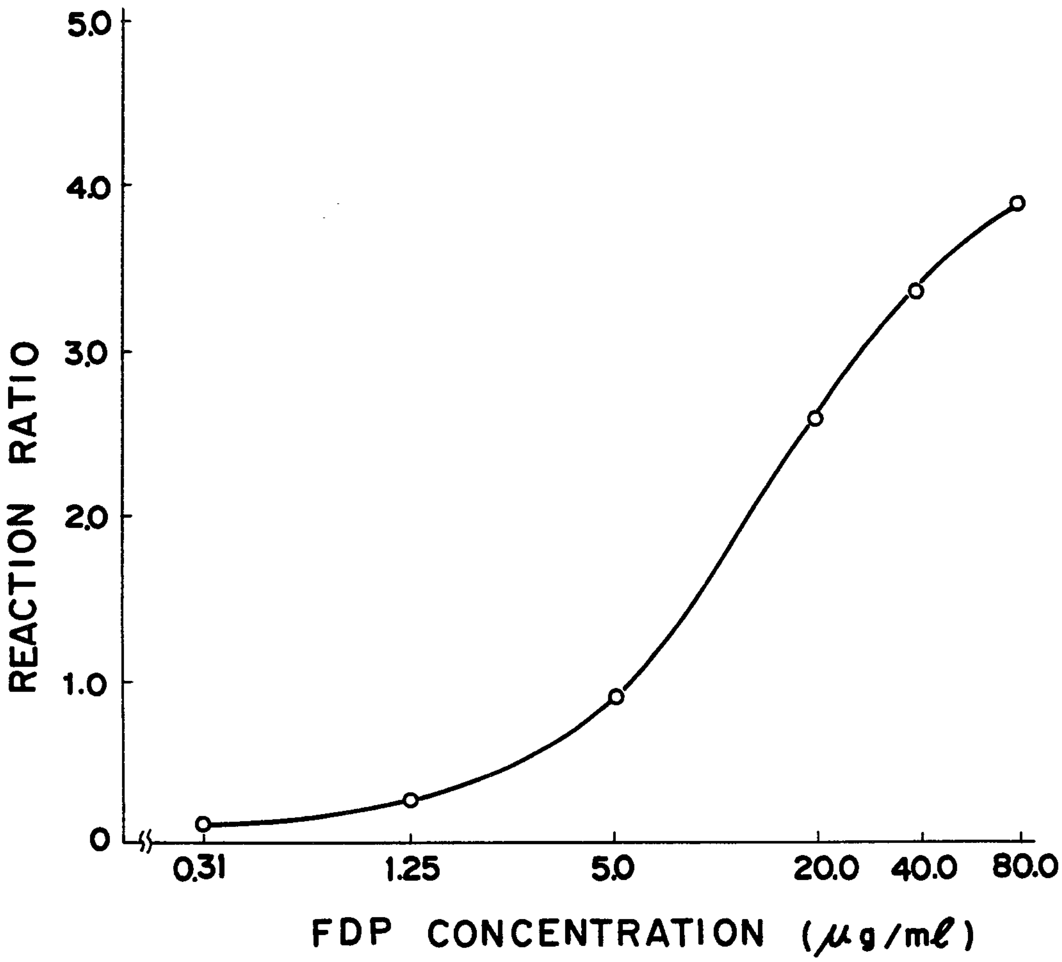
5.0
4.0
3.0
2.0
1.0
0
REACTION RATIO
0.31
1.25
5.0
20.0
40.0
80.0
FDP CONCENTRATION (μg/mℓ)

FIG. 4

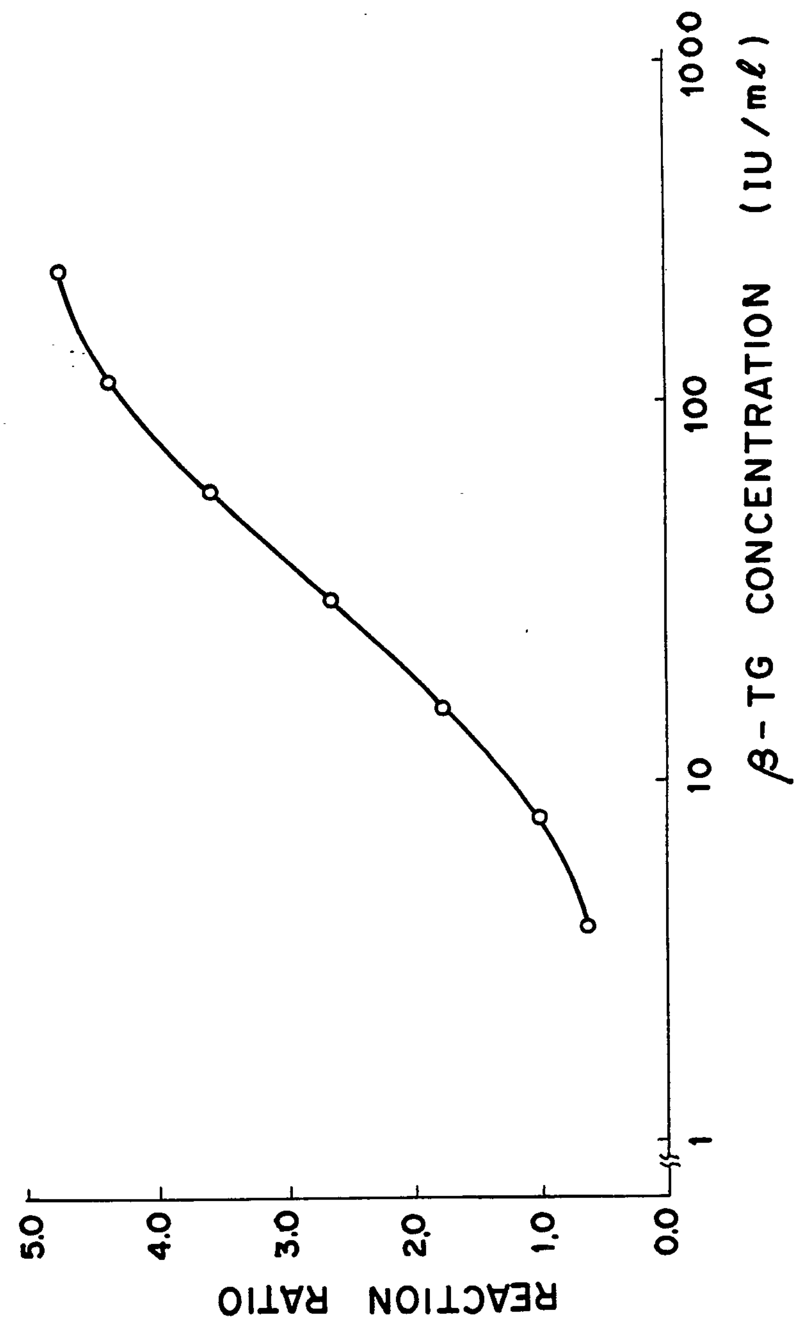
REACTION RATIO
0.0
1.0
2.0
3.0
4.0
5.0
1
10
100
1000
β - TG CONCENTRATION (IU/mℓ)